# EUROPÄISCHE PATENTSCHRIFT

(11) **EP 0 468 003 B1**
(45) Veröffentlichungstag und Bekanntmachung des Hinweises auf die Patenterteilung: **28.09.1994**
(21) Anmeldenummer: 91901671.7
(22) Anmeldetag: 24.01.1991
(51) Int. Cl.: A61K 9/50, A61K 47/36

(54) **EINKAPSELUNG VON WIRKSTOFFEN MITTELS STÄRKE**
ENCAPSULATION OF ACTIVE SUBSTANCES IN STARCH
ENCAPSULATION DE SUBSTANCES ACTIVES AU MOYEN D'AMIDON

(30) Priorität: 26.01.1990 DE 4002257
(43) Veröffentlichungstag der Anmeldung: 29.01.1992
(73) Patentinhaber: F. HOFFMANN-LA ROCHE AG, 4002 Basel (CH); Bio-tec Biologische Naturverpackungen GmbH, D-46446 Emmerich (DE)
(72) Erfinder: TOMKA, Ivan, CH-1722 Bourguillon (CH); SALA, Robert, CH-8032 Zürich (CH)
(74) Vertreter: Cottong, Norbert A.
(86) Internationale Anmeldenummer: CH9100020
(87) Internationale Veröffentlichungsnummer: WO9111178

(56) Entgegenhaltungen:
- EP-A- 0 281 513
- DE-A- 2 708 513
- GB-A- 2 021 948
- US-A- 4 755 397

## Beschreibung

Die vorliegende Erfindung befasst sich mit einem Verfahren zur Einkapselung oder zum Beschichten eines oder mehrerer Wirkstoffe in einer oder mit einer Trägersubstanz, mit einem eingekapselten oder beschichteten Wirkstoff, welcher mittels einer Trägersubstanz umhüllt ist sowie mit Anwendungen der erfindungsgemässen Verfahren.

Das Vorlegen von Wirkstoffen, wie z.B. von Pharmazeutika, Klebstoffen, Riechstoffen, Waschmittelzusätzen, Farbstoffen, Betonzusätzen, Pestiziden usw., in einer weiterverarbeitungsfähigen, einer weiterverwendbaren, transportierbaren, verkaufsfähigen usw. Form ist seit langem ein zentrales Problem. Insbesondere müssen Wirkstoffe vor Umgebungseinflüssen, wie Feuchtigkeit, UV-Strahlung, mechanischer Beanspruchung usw., geschützt werden und sollten gleichzeitig gut zu dosieren und praktisch in der Handhabung sein.

Eine Vielzahl von Prozessen, Verfahren und Techniken ist bekannt, um Wirkstoffe in einer handhabbaren Form vorzulegen, wie dies entsprechend der oben angeführten Problemstellung erforderlich ist.

Bekannt ist z.B. das Herstellen von Tabletten, wo im wesentlichen feste Wirkstoffe mittels eines Verfestigungsmittels in Tablettenform gepresst werden. Insbesondere für feuchtigkeitsempfindliche Wirkstoffe oder für die Feindosierung ist diese Handhabungsform ungeeignet.

Das Abpacken in kleine Beutel, beispielsweise bestehend aus Kunststoff, weist den Nachteil auf, dass die Verpackung bei Verwendung der Wirkstoffe, d.h. der Beutel, zunächst geöffnet werden muss, was insbesondere bei kleinen Beuteln sehr unpraktisch ist. Zudem entstehen Abfälle, falls der Beutel nicht mitverwendbar ist.

Von besonderem Interesse ist das Beschichten resp. Einkapseln von Wirkstoffen, wozu in letzterem Falle Kapseln oder Mikrokapseln hergestellt werden, welche mit dem Wirkstoff gefüllt werden. Die zur Herstellung dieser Kapseln resp. für deren Füllen üblichen Verfahren sind aber sehr aufwendig und teuer, so dass sie insbesondere bei relativ billigen Wirkstoffen kaum oder überhaupt nicht rentabel sind. Das Prinzip dieser Einkapselung ist bis heute im wesentlichen auf pharmazeutische Wirkstoffe reduziert, da diese Wirkstoffe relativ teuer sind und somit die Kosten des Einkapselungsprozesses nicht derart ins Gewicht fallen. Diese Technik ist auf vielfältige Art und Weise vorbeschrieben, wozu beispielsweise auf die nachfolgenden Schriften verwiesen wird.

In den EP-A-0 092 908, EP-PS-0 090 600, EP-A-0 118 240 und EP-A-0 304 401 wird die Herstellung von Kapseln mittels Spritzgiessens oder Tiefziehens beschrieben, wobei zur Herstellung der Kapseln hydrophile Polymere, wie Gelatine oder Stärke, vorgeschlagen werden, die für das Verarbeiten und die Herstellung der Kapseln mittels geeigneter Prozesse mindestens teilweise in thermoplastisch verarbeitbare Form gebracht werden. Es handelt sich hier ausschliesslich um die Herstellung von Kapseln grösserer Art, welche erst nach deren Herstellung mit Wirksubstanzen gefüllt resp. verschlossen werden.

In der Encyclopedia of Polymer Science and Engineering, Vol. 9, John Wilez & Sons, S. 724 ff., sind verschiedene Verfahren und Anwendungen sowie eine Vielzahl von Literaturzitaten über Herstellverfahren für die Herstellung von Mikrokapseln beschrieben. Dabei wird ein Wirkstoff, wie z.B. ein aktives Agens, ein Kernmaterial, ein Füllstoff, ein Nucleid usw., durch ein Trägermaterial, eine Beschichtung, eine Membran usw., eingekapselt. Die Grösse dieser Mikrokapseln liegt zwischen 1 und 1000 »m. Die wichtigsten Anwendungsgebiete liegen beim Herstellen von kohlenstoff-freien Kopierpapieren und bei der Mikroenkapsulierung von pharmazeutischen Wirkstoffen.

Insbesondere die Verwendung der bis heute verwendeten Verfahren für die Mikroenkapsulierung ist in der Encyclopedia of Chemical Technology von Kirk-Othmer, 3rd Edition, Vol. 15, auf den S. 487 ff. ausführlich beschrieben. In diesem Literaturzitat sei vor allem auf das Literaturregister auf den S. 492 und 493 verwiesen.

Da die Herstellung von Mikrokapseln an sich in den beiden genannten Literaturzitaten ausreichend beschrieben ist und die verschiedenen Herstellverfahren mannigfaltig und umfangreich sind, wird auf eine detaillierte Beschreibung derselben verzichtet.

Alle die oben vorgeschlagenen Verfahren resp. Techniken zum Herstellen von Kapseln resp. Mikrokapseln haben den gewichtigen Nachteil, dass sie sehr teuer sind und somit nur im Zusammenhang mit teuren Wirkstoffen, wie beispielsweise pharmazeutischen Produkten, überhaupt diskutabel sind. Diese Aussage wird im Literaturzitat von Kirk-Othmer auf S. 491 im letzten Abschnitt weiter erhärtet, indem hier angeführt wird, dass sog. "large scale" industrielle Verwendung der Herstellung von Mikrokapseln infolge hoher Kosten limitiert ist und somit nur im pharmazeutischen Bereich, der Medizin und einigen Spezialmärkten Zukunftsperspektiven besitzt.

Es ist daher eine Aufgabe der vorliegenden Erfindung, eine Methode zu entwickeln, gemäss welcher Wirkstoffe und Wirksubstanzen auf einfache und billige Art und Weise eingekapselt oder beschichtet werden können, wobei vorzugsweise die für die Einkapselung oder Beschichtung verwendete Trägersubstanz ein Material umfasst, das bei der Weiterverwendung der Wirksubstanz resp. des Wirkstoffes mitverwendet werden kann, so dass weder Abfallprodukte entstehen noch die Weiterverwendung der Wirksubstanz durch die Trägersubstanz negativ beeinflusst wird.

Bei dem erfindungsgemässen Verfahren handelt es sich um ein Verfahren zur Einkapselung oder zum Beschichten eines oder mehrerer Wirkstoffe in einer oder mit einer Trägersubstanz, welches Verfahren dadurch gekennzeichnet ist, dass als einkapselnde oder beschichtende Trägersubstanz ein Gemisch aus im wesentlichen nativer Stärke und mindestens einem die Stärke mindestens teilweise quellenden Mittel verwendet wird, das für die Einkapselung oder für das Beschichten zusammen mit dem Wirkstoff mindestens einem Emulgator gemischt wird.

Dabei wird bevorzugt, dass das quellende Mittel mindestens eine Substanz umfasst, deren Löslichkeitsparameter grösser als 15 [cal ^{1/2} cm ^{-3/2}] (=30.690 [J^{1/2}m ^{-3/2}]) ist und die beim. Mischen mit der Stärke deren Schmelzpunkt derart erniedrigt, dass die Schmelztemperatur der Stärke zusammen mit der Substanz unterhalb der Zersetzungstemperatur der Stärke liegt. Ferner ist es von Vorteil, dass das quellende Mittel im Gemisch zusammen mit der Stärke bei Temperaturen im Bereich nahe unterhalb des Schmelzpunktes des Gemisches einen Dampfdruck kleiner als 1bar aufweist. Vorzugsweise wird das quellende Mittel im wesentlichen aus Glycerin oder aus mindestens einem Aminoalkohol der nachfolgenden Formel gebildet:

[NHₓR₃₋ₓ]_{y}

mit: x = 0, 1 oder 2
mindestens einem R = R₁OH, R₂OH und/oder R₃OH,
wobei R₁, R₂, R₃ aliphatisch oder cyclo-aliphatisch, jedoch nicht aromatisch ist,
und mit y = 1, 2, 3 ... 100 mit einer Molmasse <2000.

Ein weiteres bevorzugtes quellendes Mittel ist im wesentlichen Wasser.

Die Wahl des die Stärke anquellenden oder quellenden Mittels wird vorzugsweise derart gewählt, dass der zu enkapsulierende Wirkstoff im quellenden Mittel schwach oder schwerlich löslich ist.

Vorzugsweise wird im Falle von in Wasser gut löslichen Wirkstoffen das erfindungsgemässe Verfahren so durchgeführt, dass beim Mischen der im wesentlichen nativen Stärke mit dem die Stärke quellenden Mittel wenigstens teilweise Feuchtigkeit von der Stärke abgezogen wird.

Dabei kann es bevorzugt sein, dass die wenigstens teilweise gequollene Stärke bei der Enkapsulierung oder Beschichtung des Wirkstoffes weniger als 12Gew% Wasser enthält, wobei dies bei einer weiteren bevorzugten Ausführungsvariante des erfindungsgemässen Verfahrens bis zur weitgehendsten Wasserfreiheit der gequollenen Stärke führen kann, d.h. wobei bevorzugt ist, dass die wenigstens teilweise gequollene Stärke im wesentlichen wasserfrei ist.

Im weiteren ist es auch möglich, dass der Wirkstoff selbst wenigstens teilweise das die Stärke quellende Mittel darstellt oder aber im Quellmittel löslich ist.

Als geeignete Quellungs- resp. Anlösemittel der Stärke haben sich insbesondere Glycerin und Aminoalkohole herausgestellt, wie sie im kennzeichnenden Teil des Anspruches 5 beansprucht sind. Dabei kann es sich bei den Aminoalkoholen um Monomere wie auch um Oligomere der angeführten Aminoalkohole handeln. Insbesondere als geeignet hat sich beispielsweise Triaethanolamin erwiesen.

Wie gemäss der Erfindung vorgeschlagen wird, wird dem Gemisch aus nativer Stärke und dem quellenden Mittel weiter ein Emulgator zugesetzt. Durch die Grenzflächenaktivität reichert sich dieser Emulgator an der Oberfläche der aufgequollenen Stärke an und beeinflusst die Benetzung zwischen dem Wirkstoff und der gequollenen Stärke. Durch den Emulgator wird die vollständige Benetzung der gequollenen Stärke unterstützt, wodurch die Aufnahme der Wirksubstanz durch die Stärke resp. die Beschichtung der Wirksubstanz begünstigt wird. Vorzugsweise wird vorgeschlagen, bis zu ca. 4Gew%, bezogen auf die Gesamtmischung des Emulgators, zu verwenden.

Um weiter zu gewährleisten, dass die QuellmittelStärkephase nicht eine zusammenhängende homogene Masse bildet, wird weiter vorgeschlagen, dass beim Mischen der gequollenen Stärke mit der Wirksubstanz weiter mindestens ein ölartiger Stoff zugesetzt wird, der mit der Stärke im wesentlichen kein homogenes Gemisch bilden kann, resp. wobei das Oel und die gequollene Stärke zwei getrennte Phasen bilden und die gequollenen Stärkepartikel (Stärkekörner) mit mindestens einem Oelfilm umhüllt sind oder in der Oelphase dispergiert sind.

Bevorzugt wird vorgeschlagen, dass beim Mischen der gequollenen Stärke mit dem Wirkstoff bis zu 10Gew%, vorzugsweise 5 bis 10Gew%, bezogen auf die Gesamtmischung, von einem ölartigen Stoff zugemischt wird, welcher sich nicht homogen mit der gequollenen Stärke mischen lässt. Dabei ist es wesentlich, dass der Wirkstoff einerseits in dieser öligen Phase nicht löslich ist, und zudem muss er durch die Quellmittel-Stärkephase besser benetzbar sein als durch die Oelphase, wodurch gewährleistet wird, dass die Stärke-Quellmittelphase den Wirkstoff umhüllt.

Ebenfalls, um die Bildung einer zusammenhängenden homogenen Masse zu verhindern, wird weiter vorgeschlagen, dass die native Stärke derart mindestens teilweise angequollen und mit dem Quellmittel gemischt wird, dass im wesentlichen eine Kornstruktur erhalten bleibt. Dies heisst beispielsweise, dass die Temperatur nicht derart hoch gewählt wird, dass die Körner aufgeschmolzen werden und eine homogene amorphe Stärkephase bilden. Andererseits darf die mechanische Beanspruchung der Körner (Scheren, Kneten, Mischen) nur so gross sein, dass der Wirkstoff in die Körner eingearbeitet wird, die Körner aber nicht zerstört werden. Die Strukturviskosität der Stärkekörner darf nicht eine untere Grenze unterschreiten, die in etwa bei einer Scherrate von 100 sec⁻¹ bei ca. 20 Pa/sec liegt. Die gequollenen Stärkekörner können dabei bereits erhöhte amorphe Struktur aufweisen, jedoch koagulieren die einzelnen Körner noch nicht, d.h. es entsteht noch keine homogene Schmelze. Die sog. partikuläre Phase der gequollenen Stärke muss erhalten bleiben.

So darf beispielsweise die Temperatur beim Mischen der einzelnen Komponenten bei Verwendung von 40% Glycerin als Quellmittel nicht über 170°C liegen.

Gemäss dem erfindungsgemässen Verfahren wird weiter vorgeschlagen, dass bis zu 60Gew%, vorzugsweise 30 bis 50Gew% des Quellmittels zugesetzt wird. Damit wird gewährleistet, dass die ehemals harten nativen Körner der Stärke ausreichend aufgequollen werden und damit elastische Körner bilden, in welche sich leicht die verschiedenen Wirkstoffe einkneten lassen. Dabei ist die Wahl der Zusatzmenge des Quellmittels stark vom gewählten Quellmittel abhängig, womit selbstverständlich auch mit weniger als 30% zugesetztem Quellmittel, wie beispielsweise von Wasser, wirkungsvoll Wirksubstanzen eingekapselt werden können. Setzt man jedoch mehr als 60% eines Quellmittels zu, so werden die Körner zu weich, so dass sie schon bei kleiner mechanischer Beanspruchung zerplatzen und das körnige, pulverige Material zu einer kompakten Masse zusammenkleben kann.

Je nach den geforderten Eigenschaften des Endproduktes, wie auch aufgrund von möglichen toxischen Eigenschaften des Quellmittels, kann es gegebenenfalls von Vorteil sein, das Quellmittel nach der Einkapselung resp. dem Beschichten wenigstens teilweise wieder abzuziehen.

Insbesondere bei der Verwendung von bis zu 10Gew% Zusatz eines ölartigen Stoffes liegt die Zusatzmenge des verwendeten Emulgators, vorzugsweise bei 1 bis 2Gew%, bezogen auf die Gesamtmischung.

Das erfindungsgemässe Verfahren zur Einkapselung oder zum Beschichten eines oder mehrerer Wirkstoffe in einer oder mit einer Trägersubstanz umfasst insbesondere das Mischen einer Mischung aus 20 bis 60Gew% eines Quellmittels zum Anquellen der nativen Stärke, vorzugsweise 30 bis 50Gew%, von 0,1 bis 4Gew% eines Emulgators, von 0 bis 10Gew% eines ölartigen Stoffes, und der Rest nativer Stärke in einem Mischaggregat, wie Mischer, Kneter oder Extruder, wobei das Mischen zusammen mit dem Wirkstoff, vorzugsweise bei erhöhter Temperatur, erfolgt, um so ein im wesentlichen homogenes Pulver zu erzeugen, welches den eingekapselten resp. beschichteten Wirkstoff enthält. Die mechanische Agitation im Mischer, Kneter oder Extruder bei erhöhter Temperatur erfolgt deshalb, weil der Benetzungs- und Sorptionsvorgang der gequollenen Stärke durch die Wirksubstanz infolge der hohen Viskosität der Stärke/Quellmittelphase ohne diese zusätzlichen Massnahmen nur sehr langsam erfolgt. Der Quellprozess ist auch bei Raumtemperatur durchführbar, wobei folgende Probleme auftauchen:
- Es dauert je nach Quellungsmittelgehalt bis zu 24 Stunden, bis alles Quellmittel eingezogen ist.
- Wegen schlechter Durchmischung ist die Homogenität des Produktes nicht gewährleistet (verschieden grosse Körner).

Dabei wird die Mischung zunächst auf eine Temperatur erwärmt, die unterhalb der Schmelztemperatur des Stärke/Quellmittelgemisches liegt. Anschliessend wird die Stärke mittels des Quellmittels mindestens teilweise gequollen, worauf unter Einwirkung der mechanischen Mischagitation die gequollene Stärke den Wirkstoff einhüllt resp. diesen durch Sorption aufnimmt, wobei der Emulgator und gegebenenfalls das Oel bewirken, dass statt einer zusammenhängenden Masse ein im wesentlichen homogenes Pulver entsteht.

Als Emulgatoren haben sich u.a. beispielsweise die nachfolgenden Substanzen als geeignet erwiesen:
- Lecithin,
- ein Polyoxyethylenderivat eines Sorbitanesters, wie beispielsweise Tween® von der Firma ICI,
- ein Aethylenoxidderivat von Zucker oder Zuckerester, Zuckeralkohol und/oder Zuckeralkoholester.

Als ölartiger Stoff kann beispielsweise im wesentlichen mindestens ein Triglycerid verwendet werden.

Die Dosierung der Trägersubstanz mit dem oder den Wirkstoffen wird zweckmässigerweise durch die Wahl der mittleren Korngrösse der verwendeten nativen Stärke und/oder dem Quellgrad der Stärke resp. dem Verhältnis von Anteil Stärke zu quellendem Mittel eingestellt. Die mittlere Partikelgrösse der eingekapselten Wirkstoffe kann durch die Wahl der mittleren Körngrösse der verwendeten nativen Stärke eingestellt werden. Wird beispielsweise von einem nativen Stärkekorn von Mais ausgegangen, dessen mittlere Korngrösse im Bereich von ca. 14 »m liegt, so kann ein derartiges Stärkekorn wesentlich schwächer mit einer Wirksubstanz "gefüllt" werden als beispielsweise ein natives Stärkekorn der Kartoffel, deren mittlere Korngrösse im Bereich von ca. 35 »m liegt. Generalisiert kann ausgesagt werden, dass, je grösser die mittlere Korngrösse der nativen Stärke ist, von welcher ausgegangen wird, um so mehr Wirkstoff bei gleicher Schutzwirkung beschichtet werden kann. Dabei ist allerdings zu bedenken, dass grössere Körner wohl grössere Schutzwirkung aufweisen, jedoch die Gesamtmischung der enkapsulierten Wirksubstanzen weniger homogen ist.

Da die einmal enkapsulierten Wirksubstanzen nicht gemahlen werden können, ist es daher ebenfalls wichtig, über die Korngrösse der nativen Stärke, von welcher ausgegangen wird, die Partikelgrösse des schlussendlich enkapsulierten Wirkstoffes zu steuern.

Mittels des vorab beschriebenen erfindungsgemässen Verfahrens wird ein eingekapselter oder beschichteter Wirkstoff hergestellt, welcher mittels einer Trägersubstanz umhüllt ist, wobei die Trägersubstanz für die Einkapselung resp. Beschichtung im wesentlichen aus mindestens teilweise gequollener Stärke besteht.

Der eingekapselte oder beschichtete Wirkstoff, der einen weiteren Aspekt der vorliegenden Erfindung darstellt, zeichnet sich insbesondere dadurch aus, dass zu dessen Herstellung als einkapselnde oder beschichtende Trägersubstanz ein Gemisch aus im wesentlichen nativer Stärke und mindestens einem die Stärke mindestens teilweise quellenden Mittel verwendet wird, das für die Einkapselung oder für das Beschichten zusammen mit dem Wirkstoff und mindestens einem Emulgator gemischt wird.

Das vorab beschriebene erfindungsgemässe Verfahren eignet sich insbesondere für das Einkapseln oder Beschichten von pharmazeutischen oder kosmetischen Wirkstoffen und/oder für die Herstellung von Medikamenten oder medizinischen Indikationen. Die entsprechende Anwendung des Verfahrens stellt einen weiteren Aspekt der vorliegenden Erfindung dar.

Besonders interessant ist das erfindungsgemässe Verfahren zudem zum Einkapseln von wasserlöslichen oder mit Wasser mischbaren Substanzen, insbesondere wasserlösliche Vitamine und Zitronensäure. Die entsprechenden eingekapselten oder beschichten Wirkstoffe, und die entsprechende Anwendung des erfindungsgemässen Verfahrens, sind bevorzugt.

Weiter eignet sich das Verfahren für die Einkapselung oder Beschichtung von Klebstoffen, Geschmacksstoffen, Riechstoffen, Waschmitteln, Pestiziden, Herbiziden, Farbstoffen, Kunstharzzusätzen, Baustoffadditiven, Betonzusätzen und/oder Reaktanden für die Beschichtung von kohlenstoff-freien Kopierpapieren, sowie für die Einkapselung oder Beschichtung von zähflüssigen, hochviskosen Flüssigkeiten und/oder Emulsionen, wie Aromastoffen, Farbstoffzusätzen, Kunstharzzusätzen, Baustoffadditiven und Betonzusätzen, insbesondere für die Verbesserung ihrer Verarbeitbarkeit resp. Dosierbarkeit.

Stellvertretend für diese Anwendungsbereiche sei speziell auf die Verwendung des erfindungsgemässen Verfahrens auf die Einkapselung von Betonzusätzen, wie Netzmittel, Aushärtungsregulatoren, Kunstharzzusätzen etc. verwiesen, deren Einsatz infolge ihres Vorliegens als hochviskose, zähe Flüssigkeiten schwierig ist. Dem trockenen Zement können sie nicht oder nicht dosierbar zugesetzt werden, und beim Zusetzen zum Nasszement wird ihre Reaktion sofort in Gang gesetzt.

Demgegenüber ist die trockene, pulverförmige Stärke/Wirkstoffmischung leicht dem trockenen Zement zumischbar, wobei sowohl Dosierung als auch anschliessende Lagerung des Zementes während einer längeren Zeitperiode unproblematisch ist. Wirksam werden die Zusätze je erst beim Hinzufügen des Wassers.

An dieser Stelle sei auf die Literaturzitate in bezug auf die Herstellung und Verwendung von Mikrokapseln verwiesen, welche in der Beschreibungseinleitung als Stand der Technik angeführt worden sind und in welchen umfangreiche Anwendungen für Mikrokapseln angeführt sind. In diesem Zusammenhang sei weiter auf Ullmann's Encyclopedia of Industrial Chemistry, 5th Edition, Vol. A 5, verwiesen, wo auf den S. 518 ff. ausführlich auf mögliche Additive bei der Herstellung von Beton verwiesen ist, für deren Einkapselung ebenfalls die vorab angeführten erfindungsgemässen Verfahren geeignet sind.

Anschliessend wird nun anhand der beigefügten 5 Beispiele der Einkapselung von Vitamin C die vorab beschriebene Erfindung näher erläutert.

### Enkapsulierung von Vitamin C:

### Beispiel 1:

4 kg native Kartoffelstärke werden mit 2,5 kg einer Mischung, bestehend aus den folgenden Komponenten:
- 77,6% Glycerin,
- 15,5% Triglycerin (Miglyol 812),
- 6,9% Emulgator (Tween® 80 der Firma ICI),
mit einem Knetextruder (Firma Buss, Pratteln, Schweiz) während einer Stunde extrudiert. Der Wassergehalt der Stärke betrug dabei ca. 12%. Im Knetextruder wurden folgende Prozessbedingungen gewählt:
- Wellentemperatur 110°C,
- Heizzone 1: 113°C,
- Heizzone 2: 116°C,
- Heizzone 3: 122°C,
- Drehzahl: 140 Umdrehungen/min.,
- Leistungsaufnahme: 0,6 kw.

Als Produkt entstand ein flockiges, weisses Pulver, wobei unter dem Mikroskop deutlich einzelne Körner, welche mit Glycerin gequollen sind, zu erkennen sind.

Durch Homogenisieren im Kneter wird eine mittlere Teilchengrösse von ca. 100»m erreicht. Durch das verwendete Oel wird ein Zusammenkleben der Stärkepolymerkugeln verhindert. Als Polymer, bei welchem es sich im vorliegenden Beispiel um Kartoffelstärke handelt, können selbstverständlich auch andere Stärken verwendet werden.

Dem auf obige Weise erhaltenen Pulver wurde bei einem zweiten Durchgang im Kneter 20% Vitamin C zugegeben und mit den gleichen Parametern eingeknetet. Das Resultat ist ebenfalls unter dem Mikroskop gut zu sehen. Der grösste Teil der Vitamin C-Kristalle ist in den elastischen Körpern eingebettet. Wenn man beispielsweise die Körner flachdrückt, erkennt man unter dem Polarisationsmikroskop darin die eingebetteten Vitamin C-Kristalle.

In diese elastischen Körner können auch andere Wirkstoffe eingearbeitet werden. Einzige Bedingung ist, dass eine gewisse Affinität des Wirkstoffes zur polaren Glycerinstärke vorhanden sein muss, d.h. der Wirkstoff muss durch die Glycerinstärkemasse benetzbar sein.

### Beispiel 2:

Gleiche Zusammensetzung wie unter 1), aber über Nacht stehengelassen (nicht extrudiert). 12 Stunden später hat man ebenfalls ein gequollenes Pulver, in welches Vitamin C eingeknetet wird. Dieser Versuch zeigt, dass die Erhöhung der Temperatur nur die Quellung beschleunigt, nicht aber Bedingung ist, dass die Quellung erfolgt. Das Vitamin C wird, wie im ersten Beispiel, mit dem Busskneter eingearbeitet.

### Beispiel 3:

In einem Brabender Laborkneter (70g-Batches) werden 40g Stärke (Wassergehalt: 12%) und 25g der Mischung von Beispiel 1) ca. 2 Minuten geknetet und anschliesend 16g (20%) Vitamin C zugegeben und weitere 2 Minuten geknetet. Das Vitamin C wird zu über 90% in die Stärkekörner eingeknetet. Weitere Versuche mit 30% bzw. 40% Vitamin C zeigen ebenfalls positive Resultate, nur der Prozentsatz an nicht eingekapseltem Vitamin C steigt langsam an.

### Beispiel 4:

16kg native Kartoffelstärke (Wassergehalt: 5%) werden mit 10,8kg der gleichen Mischung, wie oben in einem gleichläufigen Doppelwellenextruder bei 150°, gequollen. Diesem Pulver werden 20% Vitamin C in einem Taumelmischer zugegeben und anschliessend bei 140°C nochmals extrudiert, um das Vitamin C einzukneten.

### Beispiel 5:

Gleiche Bedingungen wie unter Beispiel 4), nur in einem Schritt durchgeführt. Die Schneckenkonfiguration des Extruders wird so modifiziert, dass beim Einzug Stärke und Emulsion gemischt und zu Pulver gequollen werden und in der Mitte des Extruders direkt Vitamin C zugegeben wird. Am Schluss erhält man ein feines Pulver von eingekapseltem Vitamin C, welches nicht weiter nachbehandelt (Mahlen, Sieben usw.) werden muss.

Die oben angeführten Beispiele der Einkapselung von Vitamin C mittels Glycerin/Stärke dienen einzig der Erläuterung des erfindungsgemässen Verfahrens, welche selbstverständlich, wie in der Beschreibung ausführlich-offenbart, in verschiedenster Art und Weise abgeändert werden können.

So eignet sich das Verfahren auch zum Einkapseln anderer Vitamine, pharmazeutischen oder kosmetischen Wirkstoffen, wobei besonders interessant das Einkapseln oder Beschichten von resp. mit wasserlöslichen oder mit wassermischbaren Substanzen ist, wie insbesondere wasserlöslichen Vitaminen und u.a. mit Zitronensäure. Stellvertretend sind in nachfolgendem Beispiel 6 einige Wirksubstanzen gemäss dem erfindungsgemässen Verfahren verarbeitet worden, wobei selbstverständlich die Liste möglicher Wirksubstanzen auf vielfältigste Weise erweiterbar ist.

### Beispiel 6:

Wie oben erwähnt, wurden verschiedene Wirkstoffe in gequollene resp. thermoplastisch verarbeitbare Stärke, beinhaltend ca. 27% bis 30% Glycerin, eingearbeitet. Die praktische Durchführung erfolgte bei 140°C in einem sog. Brabender-Kneter, wobei analog den vorab beschriebenen Beispielen 70g-Batches eingesetzt wurden.

Als Wirkstoffe wurden verwendet Riboflavin (Vitamin B₂), Pyridoxin (Vitamin B₆), Nicotinsäure, Methionin, Zitronensäure, Thiamin-NO₃ (Nitrat) und Lysin-HCl.

Grundsätzlich können die beschriebenen Wirkstoffe analog Vitamin C, wie im Beispiel 5 beschrieben, in die gequollene Stärke eingearbeitet werden, um ein feines Pulver von eingekapseltem Wirkstoff zu bilden.

Da sich die erwähnten Wirkstoffe, wie Füllstoffe bei technischen Polymeren, verhalten, ist auch ein Einextrudieren dieser Wirkstoffe in gequollene Stärkeschmelze möglich, wobei in der nachfolgenden Tabelle I die entsprechende Schmelze, die Fliessfähigkeit der Schmelze und die anschliessende Schneidbarkeit untersucht resp. aufgelistet sind.

**Tabelle I**

| Stoff | Gew% Wirkst. | Schmelze | Zersetzg. | Klebr.k. | Extr. |
|---|---|---|---|---|---|
| Riboflavin(B₂) | 20% | ++,gelb | keine | tr,h,sp | ++ |
| Pyridoxin(B₆) | 30% | ++ | möglich | gummig | + |
| Nicotinsäure | 20% | ++,zäh | keine | tr,++ | ++ |
| Methionin | 20% | ++,braun | möglich | tr,++ | + |
| Zitronensäure | 20% | ++ | keine | tr,++ | ++ |
| Thiamin-NO₃ | 20% | ++,gelb | möglich | tr,++ | + |
| Lysin-HCl | 20% | gummi | keine | kl,+ | + |
| Legende: + gut ++ sehr gut tr trocken h hart sp spröde kl klebrig | | | | | |

Die in Tabelle I dargestellten Resultate haben insofern nur beschränkte Gültigkeit, da beim Einsatz von zusätzlichen Additiven in der Schmelze die Aussagen ganz anders aussehen können.

Wie bereits vorab in der Beschreibung erwähnt, ist das erfindungsgemässe Verfahren nicht nur für die Beschichtung von medizinischen, kosmetischen oder pharmazeutischen Wirkstoffen geeignet, sondern auch für das Einkapseln und Beschichten von Farbstoffadditiven, Lebensmittelzusätzen, Betonadditiven, Waschmitteladditiven usw.

Stellvertretend für diese fast unbeschränkte Gruppe von möglichen Additiven und Zusätzen soll nachfolgend die Erfindung beispielsweise anhand eines Lebensmittelzusatzes und von Betonadditiven näher erläutert werden.

### Beispiel 7: Zitronenaroma

Bis zu 40Gew% Zitronenaroma werden kalt mit trockener Stärke geknetet. Die Zitronenaroma-Emulsion enthält sehr flüchtige Komponenten, welche bei Erhöhung der Temperatur verdampfen würden. Die Emulsion zieht innerhalb von 2 Min. in die Stärke ein, und es entsteht ein gelbes Pulver, das weniger intensiv nach Zitrone duftet als die Emulsion selbst. Wird das Pulver erwärmt, so fängt es sofort an zu kleben und bildet perlengraue Knollen.

Der Vorteil des so hergestellten Pulvers gegenüber der Emulsion selbst liegt darin, dass das Pulver sehr einfach weiterverarbeitet werden kann und vor allem sehr gut dosierbar ist. Die bisher benutzte Emulsion dagegen ist nicht stabil, und sie muss immer wieder angerührt werden. Zudem trocknet die Emulsion schnell aus und verliert das Aroma, wenn sie offen stehen gelassen wird. Demgegenüber verliert das Pulver das Zitronenaroma praktisch nicht.

### Beispiel 8: Betonadditive

Bei den Betonadditiven handelt es sich meistens um hochviskose, zähflüssige Substanzen, die nur schlecht dosiert werden können und insbesondere dem trockenen Zement nicht homogen beigemischt werden können. Werden die Betonadditive erst dem bereits angemachten wässrigen Zement zugesetzt, so wird ihre Wirksamkeit sofort in Gang gesetzt, und der Zement muss unmittelbar verarbeitet werden.

Die im Zusammenhang mit dem erfindungsgemässen Verfahren untersuchten Betonadditive sind
- Rheobuild 1000, ein Naphtalinsulfonat (NA-Salz), SO₄-NA,
- Rheobuild 2000, ein Melaminderivat der nachfolgenden Formel: 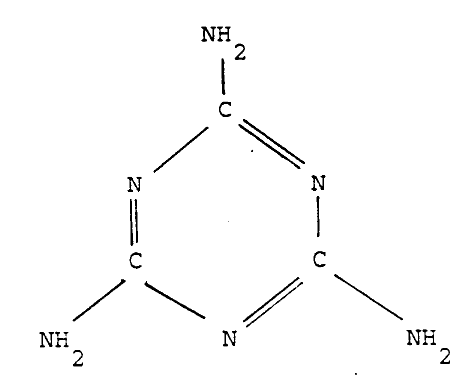 wobei 1 oder 2 -NH₂ gegen -Cl oder -OH oder Alkyl ausgetauscht sein kann,
- Rheobuild SV 87056, eine Polykarboxyl-Verbindung auf der Basis von Polyacrylsäure-NA-Salz resp. ein Mischpolymer aus Acrylsäureestern und freien Acrylsäuren und deren NA-Salz,
- Beckopox EH 623, ein Aminepoxihärter, sowie
- Rütapox EH 4000, ein Epoxidharz.

Alle die angeführten Betonadditiv-Wirkstoffe sind Markenprodukte und erhältlich bei der Firma Masterbuilders Technologies Europe AG, Ifangstrasse 11 in 8952 Schlieren/CH.

Diese flüssigen Betonadditive lassen sich gut ohne zusätzliches Quellmittel, wie Glycerin oder Aminoaethanol, mit trockener Stärke kneten. Glycerin könnte im übrigen nicht verwendet werden wegen seiner Unverträglichkeit mit Beton. Offensichtlich bildet in den vorliegenden Fällen der Wirkstoff selbst auch noch das Quellmittel für das Anquellen der Stärke. Nach max. 2 Min., unter eventuellem Heizen, sind die Flüssigkeiten in der Stärke eingezogen. wobei dies gelegentlich unter Wärmeentwicklung geschehen kann.

Falls zusätzlich als Quellmittel Aminopropanol resp. Aethanol eingesetzt wird, sollte die Quellung kalt gestartet werden, damit die Stärke nicht verkleistert, oder aber es muss ein Emulgatoröl zugesetzt werden wie bei den vorab beschriebenen Beispielen in bezug beispielsweise auf Vitamin C.

In der nachfolgenden Tabelle II sind die Wirkstoffe gemäss Beispiel 7 und 8 tabellarisch dargestellt, wobei ebenfalls deren Verhalten beim Quellen resp. Einziehen in die Stärke näher untersucht worden ist.

**Tabelle II**

| Art | Quellmittel f. Stärke | Löslich in Aminoethanol | Mischgasverhältn. | Prozessbeschrieb |
|---|---|---|---|---|
| Zitronenaroma | ja, nur kalt (20°C) | | 30-40% | nur kalt geknetet |
| Rheobuild 1000 | nein | ja | max.30% | heiss geknetet |
| Rheobuild 2000 | ja, aber kalt | | max.30% | heiss geknetet |
| Rheobuild SV 87056 | ja, heizen (≃ 140°C) | ja | max.30% | heiss geknetet |
| Beckopox EH 623 | ja, gut | ja | 20% | heiss geknetet |
| Rütapox EH 4000 | ja | ja | 20% | heiss geknetet |

Die in den Beispielen 1 bis 8 beschriebenen Wirkstoffe stehen stellvertretend für eine fast unbegrenzte

Liste von möglichen Wirkstoffen und Substanzen, welche gemäss dem erfindungsgemässen Verfahren eingekapselt resp. beschichtet werden können. Auch das Einkapselungs- und Beschichtungsverfahren selbst kann in x-beliebiger Art und Weise abgeändert resp. modifiziert werden.

Wesentlich für das erfindungsgemässe Verfahren ist die Verwendung von nativer Stärke, eines die native Stärke mindestens teilweise anquellenden Quellmittels sowie eines Emulgators, wobei die Wirksubstanz durch die Quellmittel/Stärkephase benetzbar sein muss. Dabei ist es durchaus möglich, dass der Wirkstoff selbst als Quellmittel dient. Vorzugsweise wird weiter ein ölartiger Stoff verwendet.

## Patentansprüche

1. Verfahren zur Einkapselung oder zum Beschichten eines oder mehrerer Wirkstoffe in einer oder mit einer Trägersubstanz, dadurch gekennzeichnet, dass als einkapselnde oder beschichtende Trägersubstanz ein Gemisch aus im wesentlichen nativer Stärke und mindestens einem die Stärke mindestens teilweise quellenden Mittel verwendet wird, das für die Einkapselung oder für das Beschichten zusammen mit dem Wirkstoff und mindestens einem Emulgator gemischt wird.

2. Verfahren nach Anspruch 1, dadurch gekennzeichnet, dass das quellende Mittel mindestens eine Substanz umfasst deren Löslichkeitsparameter grösser als 15 [cal ^{1/2}cm ^{-3/2}] (= 30.690 J^{1/2}m ^{-3/2}) ist und die beim Mischen mit der Stärke deren Schmelzpunkt derart erniedrigt, dass die Schmelztemperatur der Stärke zusammen mit der Substanz unterhalb der Zersetzungstemperatur der Stärke liegt.

3. Verfahren nach Anspruch 1 oder 2, dadurch gekennzeichnet, dass das quellende Mittel im Gemisch zusammen mit der Stärke bei Temperaturen im Bereich nahe unterhalb des Schmelzpunktes des Gemisches einen Dampfdruck kleiner als 1bar aufweist.

4. Verfahren nach einem der Ansprüche 1 bis 3, dadurch gekennzeichnet, dass das quellende Mittel im wesentlichen aus Glycerin gebildet wird.

5. Verfahren nach einem der Ansprüche 1 bis 3, dadurch gekennzeichnet, dass das quellende Mittel im wesentlichen aus mindestens einem Aminoalkohol der nachfolgenden Formel gebildet wird:
[NHₓR₃₋ₓ]_{y}
mit: x = 0, 1 oder 2
mindestens einem R = R₁OH, R₂OH und/oder R₃OH,
wobei R₁, R₂, R₃ aliphatisch oder cyclo-aliphatisch, jedoch nicht aromatisch ist,
und mit y = 1, 2, 3 ... 100 mit einer Molmasse <2000.

6. Verfahren nach einem der Ansprüche 1 bis 5, dadurch gekennzeichnet, dass im Falle von in Wasser gut löslichen Wirkstoffen beim Mischen der im wesentlichen nativen Stärke mit dem die Stärke quellenden Mittel wenigstens teilweise Feuchtigkeit von der Stärke abgezogen wird.

7. Verfahren nach einem der Ansprüche 1 bis 6, dadurch gekennzeichnet, dass die wenigstens teilweise gequollene Stärke bei der Einkapselung oder Beschichtung des Wirkstoffes weniger als 12Gew% Wasser enthält.

8. Verfahren nach Anspruch 7, dadurch gekennzeichnet, dass die wenigstens teilweise gequollene Stärke im wesentlichen wasserfrei ist.

9. Verfahren nach Anspruche 1 oder 2, dadurch gekennzeichnet, dass das quellende Mittel im wesentlichen Wasser ist.

10. Verfahren nach einem der Ansprüche 1 bis 9, dadurch gekennzeichnet, dass der oder die Wirkstoffe wenigstens teilweise das die Stärke quellende Mittel darstellt resp. darstellen oder in quellenden Mittel löslich ist resp. sind.

11. Verfahren nach einem der Ansprüche 1 bis 10, dadurch gekennzeichnet, dass von 0,1 bis 4Gew%, bezogen auf die Gesamtmischung, des Emulgators verwendet wird.

12. Verfahren nach einem der Ansprüche 1 bis 11, dadurch gekennzeichnet, dass beim Mischen der gequollenen Stärke mit dem Wirkstoff weiter mindestens ein ölartiger Stoff zugesetzt wird, der mit der Stärke im wesentlichen kein homogenes Gemisch bilden kann, resp. wobei das Oel und die gequollene Stärke zwei getrennte Phasen bilden und die gequollenen Stärkepartikel individuell mit mindestens einem Oelfilm umhüllt sind oder in der Oelphase dispergiert sind.

13. Verfahren nach Anspruch 12, dadurch gekennzeichnet, dass beim Mischen der gequollenen Stärke mit dem Wirkstoff bis zu 10Gew%, vorzugsweise 5 bis 10Gew%, bezogen auf die Gesamtmischung, von einem ölartigen Stoff zugemischt wird, welcher sich nicht homogen mit der gequollenen Stärke mischen lässt.

14. Verfahren nach einem der Ansprüche 1 bis 13, dadurch gekennzeichnet, dass die native Stärke derart mindestens teilweise angequollen und mit dem Quellmittel gemischt wird, dass im wesentlichen eine Kornstruktur erhalten bleibt.

15. Verfahren nach einem der Ansprüche 1 bis 14, dadurch gekennzeichnet, dass bis zu 60Gew%, vorzugsweise 30 bis 50Gew%, des Quellmittels zugesetzt wird.

16. Verfahren nach einem der Ansprüche 1 bis 15, dadurch gekennzeichnet, dass das Quellmittel nach der Einkapselung resp. dem Beschichten wenigstens teilweise wieder abgezogen wird.

17. Verfahren nach einem der Ansprüche 11 bis 16, dadurch gekennzeichnet, dass 1 bis 2Gew%, bezogen auf die Gesamtmischung, des Emulgators verwendet wird.

18. Verfahren nach einem der Ansprüche 1 bis 17, dadurch gekennzeichnet, dass eine Mischung aus:
- 20 bis 60Gew% eines Quellmittels zum Anquellen der nativen Stärke, vorzugsweise 30 bis 50Gew%,
- 0,1 bis 4Gew% eines Emulgators,
- 0 bis 10Gew% eines ölartigen Stoffes und
- der Rest native Stärke
in einem Mischaggregat, wie Mischer, Kneter oder Extruder, zusammen mit dem Wirkstoff, vorzugsweise bei erhöhter Temperatur, gemischt wird, um ein im wesentlichen homogenes Pulver zu erzeugen, welches den eingekapselten resp. beschichteten Wirkstoff enthält.

19. Verfahren nach Anspruch 18, dadurch gekennzeichnet, dass die Mischung zunächst auf eine Temperatur erwärmt wird, die unterhalb der Schmelztemperatur des Stärke/Quellmittelgemisches liegt, anschliessend die Stärke mittels des Quellmittels mindestens teilweise gequollen wird, worauf unter Einwirkung der mechanischen Mischagitation die gequollene Stärke den Wirkstoff einhüllt resp. diesen durch Sorption aufnimmt, wobei der Emulgator und gegebenenfalls das Oel bewirken, dass statt einer zusammenhängenden Masse ein im wesentlichen homogenes Pulver entsteht.

20. Verfahren nach einem der Ansprüche 1 bis 19, dadurch gekennzeichnet, dass als Emulgator mindestens eine der nachfolgenden Substanzen gewählt wird:
- Lecithin,
- ein Polyoxyethylenderivat eines Sorbitanesters
- ein Aethylenoxidderivat von Zucker oder Zuckerester, Zuckeralkohol und/oder Zuckeralkoholester.

21. Verfahren nach einem der Ansprüche 12 bis 20, dadurch gekennzeichnet, dass als ölartiger Stoff im wesentlichen mindestens ein Triglycerid verwendet wird.

22. Verfahren nach einem der Ansprüche 1 bis 21, dadurch gekennzeichnet, dass die Dosierung der Trägersubstanz mit dem oder den Wirkstoffen durch die Wahl der mittleren Korngrösse der verwendeten nativen Stärke und/oder den Quellgrad der Stärke resp. dem Verhältnis Anteil Stärke zu quellendem Mittel im Gemisch eingestellt wird.

23. Verfahren nach einem der Ansprüche 1 bis 22, dadurch gekennzeichnet, dass die mittlere Partikelgrösse der eingekapselten Wirkstoffe durch die Wahl der mittleren Korngrösse der verwendeten nativen Stärke eingestellt wird.

24. Eingekapselter oder beschichteter Wirkstoff, dadurch gekennzeichnet, dass zu dessen Herstellung als einkapselnde oder beschichtende Trägersubstanz ein Gemisch aus im wesentlichen nativer Stärke und mindestens einem die Stärke mindestens teilweise quellenden Mittel verwendet wird, das für die Einkapselung oder für das Beschichten zusammen mit dem Wirkstoff und mindestens einem Emulgator gemischt wird.

25. Eingekapselter oder beschichteter Wirkstoff nach Anspruch 24, dadurch gekennzeichnet, dass der Wirkstoff eine wasserlösliche oder mit Wasser mischbare Substanz ist, wie insbesondere ein wasserlösliches Vitamin oder Zitronensäure.

26. Anwendung des Verfahrens nach einem der Ansprüche 1 bis 23 für das Einkapseln oder Beschichten von pharmazeutischen oder kosmetischen Wirkstoffen und/oder für die Herstellung von Medikamenten oder medizinischen Indikationen.

27. Anwendung des Verfahrens nach einem der Ansprüche 1 bis 23 für das Einkapseln oder Beschichten von in Wasser löslichen oder mit Wasser mischbaren Substanzen, wie insbesondere wasserlöslichen Vitaminen und/oder Zitronensäure.

28. Anwendung des Verfahrens nach einem der Ansprüche 1 bis 23 für die Einkapselung oder Beschichtung von Klebstoffen, Geschmacksstoffen, Riechstoffen, Waschmitteln, Pestiziden, Herbiziden, Farbstoffen, Kunstharzzusätzen, Baustoffadditiven, Betonzusätzen und/oder Reaktanden für die Beschichtung von kohlenstofffreien Kopierpapieren.

29. Anwendung des Verfahrens nach einem der Ansprüche 1 bis 23 für die Einkapselung oder Beschichtung von zähflüssigen, hochviskosen Flüssigkeiten und/oder Emulsionen, wie Aromastoffen, Farbstoffzusätzen, Kunstharzzusätzen, Baustoffadditiven, Betonzusätzen etc., insbesondere für die Verbesserung ihrer Verarbeitbarkeit resp. Dosierbarkeit.

## Claims

1. A method for encapsulating or for coating one or more active compounds in a or with a carrier substance, characterized in that a mixture of essentially native starch and at least one agent at least partially swelling the starch is used as the encapsulating or coating carrier substance and for the encapsulation or for the coating is mixed together with the active compound and at least one emulsifier.

2. A method according to claim 1, characterized in that the swelling agent includes at least one substance whose solubility parameter is greater than 15 [cal^{1/2} cm^{-3/2}] (=30,690 J^{1/2}m^{-3/2}) and which on mixing with the starch reduces its melting point in such a way that the melting temperature of the starch together with the substance is below the decomposition temperature of the starch.

3. A method according to claim 1 or 2, characterized in that the swelling agent in the mixture together with the starch has a vapour pressure of less than 1 bar at temperatures in the range closely below the melting point of the mixture.

4. A method according to any one of claims 1 to 3, characterized in that the swelling agent is essentially formed from glycerol.

5. A method according to any one of claims 1 to 3, characterized in that the swelling agent is essentially formed from at least one aminoalcohol of the following formula:
[NHₓR₃₋ₓ]_{y}
with: x = 0, 1 or 2
at least one R = R₁OH, R₂OH and/or R₃OH,
where R₁, R₂ or R₃ is aliphatic or cyclo-aliphatic, but not aromatic,
and where Y = 1, 2, 3 ... 100 with a molar mass <2000.

6. A method according to any one of claims 1 to 5, characterized in that, in the case of active compounds having good solubility in water, on mixing the essentially native starch with the agent swelling the starch moisture is at least partially removed from the starch.

7. A method according to any one of claims 1 to 6, characterized in that the at least partially swollen starch contains less than 12 wt.% of water during the encapsulation or coating of the active compound.

8. A method according to claim 7, characterized in that the at least partially swollen starch is essentially anhydrous.

9. A method according to claim 1 or 2, characterized in that the swelling agent is essentially water.

10. A method according to any one of claims 1 to 9, characterized in that the active compound(s) is/are at least partially the agent swelling the starch or is/are soluble in the swelling agent.

11. A method according to any one of claims 1 to 10, characterized in that from 0.1 to 4 wt.% of the emulsifier, based on the total mixture, is used.

12. A method according to any one of claims 1 to 11, characterized in that on mixing the swollen starch with the active compound additionally at least one oily substance is added, which is essentially unable to form a homogeneous mixture with the starch, or in which the oil and the swollen starch form two separate phases and the swollen starch particles are covered individually with at least one oil film or are dispersed in the oil phase.

13. A method according to claim 12, characterized in that on mixing the swollen starch with the active compound up to 10 wt.%, preferably 5 to 10 wt.%, based on the total mixture, of an oily substance is admixed, which does not allow itself to be homogeneously mixed with the swollen starch.

14. A method according to any one of claims 1 to 13, characterized in that the native starch is at least partially swollen and mixed with the swelling agent in such a way that a grain structure is essentially retained.

15. A method according to any one of claims 1 to 14, characterized in that up to 60 wt.%, preferably 30 to 50 wt.%, of the swelling agent is added.

16. A method according to any one of claims 1 to 15, characterized in that the swelling agent is removed at least partially after the encapsulation or coating.

17. A method according to any one of claims 11 to 16, characterized in that 1 to 2 wt.%, based on the total mixture, of the emulsifier is used.

18. A method according to any one of claims 1 to 17, characterized in that a mixture of:
- 20 to 60 wt.% of a swelling agent for initiating the swelling of the native starch, preferably 30 to 50 wt.%,
- 0.1 to 4 wt.% of an emulsifier,
- 0 to 10 wt.% of an oily substance and the remainder of native starch
is mixed in a mixing apparatus, such as a mixer, kneader or extruder, together with the active compound, preferably at an elevated temperature, in order to produce an essentially homogenous powder which contains the encapsulated or coated active compound.

19. A method according to claim 18, characterized in that the mixture is first heated to a temperature which is below the melting temperature of the starch/swelling agent mixture, then the starch is swollen at least partially by the swelling agent, whereupon under the action of the mechanical agitation the swollen starch coats the active compound or takes this up by sorption, the emulsifier and possibly the oil causing an essentially homogenous powder to be formed instead of a coherent material.

20. A method according to any one of claims 1 to 19, characterized in that at least one of the following substances is chosen as the emulsifier:
- lecithin,
- a polyoxyethylene derivative of a sorbitan ester,
- an ethylene oxide derivative of a sugar or sugar ester, sugar alcohol and/or sugar alcohol ester.

21. A method according to any one of claims 12 to 20, characterized in that essentially at least one triglyceride is used as the oily substance.

22. A method according to any one of claims 1 to 21, characterized in that the metering of the carrier substance with the active compound(s) is adjusted by the choice of the mean particle size of the native starch used and/or the degree of swelling of the starch or the ratio of starch content to swelling agent in the mixture.

23. A method according to any one of claims 1 to 22, characterized in that the mean particle size of the encapsulated active compounds is adjusted by the choice of the mean particle size of the native starch used.

24. An encapsulated or coated active compound, characterized in that the encapsulating or coating carrier substance used for its production is a mixture of essentially native starch and at least one agent at least partially swelling the starch and which, for encapsulation or for coating, is mixed together with the active compound and at least one emulsifier.

25. An encapsulated or coated active compound according to claim 24, characterized in that the active compound is a water-soluble or water-miscible substance, such as especially a water-soluble vitamin or citric acid.

26. The use of the method according to any one of claims 1 to 23 for the encapsulation or coating of pharmaceutical or cosmetic active compounds and/or for the production of medicaments or medicinal indications.

27. The use of the method according to any one of claims 1 to 23 for the encapsulation or coating of water-soluble or water-miscible substances, such as especially water-soluble vitamins and/or citric acid.

28. The use of the method according to any one of claims 1 to 23 for the encapsulation or coating of adhesives, flavorants, odorants, detergents, pesticides, herbicides, dyes, synthetic resin additives, building material additives, concrete additives and/or reactants for the coating of carbon-free copying papers.

29. The use of the method according to any one of claims 1 to 23 for the encapsulation or coating of viscous and highly viscous liquids and/or emulsions, such as flavorants, dye additives, synthetic resin additives, building material additives, concrete additives etc., especially for improving their processibility or meterability.

## Revendications

1. Procédé pour encapsuler une ou plusieurs substances actives dans une substance véhicule ou pour revêtir une ou plusieurs substances actives d'une substance véhicule, caractérisé en ce que la substance véhicule utilisée pour l'encapsulation ou le revêtement est un mélange d'amidon essentiellement naturel et d'au moins un agent gonflant, en partie au moins, l'amidon, qu'on mélange avec la substance active et au moins un agent émulsionnant pour l'encapsulation ou pour le revêtement.

2. Procédé selon la revendication 1, caractérisé en ce que l'agent gonflant comprend au moins une substance dont le paramètre de solubilité est supérieur à 15 [cal^{1/2} cm ^{-3/2}] (= 30,690 J^{1/2}m^{-3/2}) et qui, au mélange avec l'amidon, abaisse son point de fusion au point que la température de fusion de l'amidon mélangé à cette substance est inférieure à la température de décomposition de l'amidon.

3. Procédé selon revendication 1 ou 2, caractérisé en ce que l'agent gonflant en mélange avec l'amidon a une pression de vapeur inférieure à 1 bar dans l'intervalle de température situé immédiatement au-dessous du point de tusion du mélange.

4. Procédé selon une des revendications 1 à 3, caractérisé en ce que l'agent gonflant consiste essentiellement en glycérol.

5. Procédé selon une des revendications 1 à 3, caractérisé en ce que l'agent gonflant consiste essentiellement en au moins un aminoalcool de formule :
[NHₓR₃₋ₓ]_{y}
dans laquelle x = 0, 1 ou 2
l'un au moins des symboles R représente R₁OH, R₂OH et/ou R₃OH
R₁, R₂, R₃ représentant des radicaux aliphatiques ou cycloatiphatiques mais non aromatiques,
et y = 1, 2, 3 ... 100, la masse moléculaire étant inférieure à 2 000.

6. Procédé selon une des revendications 1 à 5, caractérisé en ce que, dans le cas de substances actives bien solubles dans l'eau, on élimine en partie au moins l'humidité de l'amidon essentiellement naturel au mélange de cet amidon avec l'agent gonflant

7. Procédé selon une des revendications 1 à 6, caractérisé en ce que l'amidon gonflé en partie au moins contient moins de 12 % en poids d'eau à l'encapsulation ou au revêtement de la substance active.

8. Procédé selon revendication 7, caractérisé en ce que l'amidon gonflé en partie au moins est essentiellement anhydre.

9. Procédé selon la revendication 1 ou 2, caractérisé en ce que l'agent gonflant consiste essentiellement en eau.

10. Procédé selon une des revendications 1 à 9, caractérise en ce que la ou les substances actives constituent en partie au moins l'agent gonflant l'amidon ou sont solubles dans l'agent gonflant.

11. Procédé selon une des revendications 1 à 10, caractérisé en ce que l'on utilise de 0,1 à 4 % de l'agent émulsionnant par rapport au poids du mélange total.

12. Procédé selon une des revendications 1 à 11, caractérisé en ce que, au mélange de l'amidon gonflé avec la substance active, on ajoute encore au moins une substance de nature huileuse qui est essentiellement incapable de former avec l'amidon un mélange homogène, c'est-à-dire l'huile et l'amidon gonflé forment deux phases séparées et les particules d'amidon gonflé sont enrobées individuellement par au moins une pellicule d'huile ou sont dispersées dans la phase huileuse.

13. Procédé selon revendication 12, caractérisé en ce que, au mélange de l'amidon gonflé avec la substance active, on ajoute et on mélange jusqu'à 10 % en poids, de préférence de 5 à 10 % en poids, par rapport au mélange total, d'une substance de nature huileuse qui ne forme pas de mélange homogène avec l'amidon gonflé.

14. Procédé selon une des revendications 1 à 13, caractérisé en ce que l'amidon naturel est gonflé en partie au moins et mélangé avec l'agent gonflant dans des conditions elles qu'il subsiste essentiellement une structure granuleuse.

15. Procédé selon une des revendications 1 à 14, caractérisé en ce que l'on ajoute jusqu'à 60 % en poids, de préférence de 30 à 50 % en poids de l'agent gonflant.

16. Procédé selon une des revendications 1 à 15, caractérisé en ce que, après l'encapsulation ou le revêtement, l'agent gonflant est éliminé en partie au moins.

17. Procédé selon une des revendications 11 à 16, caractérisé en ce que l'on utilise l'agent émulsionnant en proportions de 1 à 2 % du poids du mélange total.

18. Procédé selon une des revendications 1 à 17, caractérisé en ce que l'on mélange dans un appareil de mélange tel qu'un mélangeur, un malaxeur ou une extrudeuse, avec la substance active, de préférence à chaud, un mélange de :
- 20 à 60 % en poids d'un agent gonflant servant à gonfler l'amidon naturel, de préférence 30 à 50 % en poids,
- 0,1 à 4 % en poids d'un agent émulsionnant,
- 0 à 10 % en poids d'une substance de nature huileuse et
- le reste de l'amidon naturel,
en vue de former une poudre essentiellement homogène qui contient la substance active à l'état encapsulé ou revêtu.

19. Procédé selon revendication 18, caractérisé en ce que le mélange est d'abord chauffé à une température inférieure à la température de fusion du mélange amidon/agent gonflant, l'amidon est ensuite gonflé en partie au moins sous l'action de l'agent gonflant, de sorte que, sous l'action de l'agitation mécanique de mélange, l'amidon gonflé enrobe la substance active ou absorbe cette substance active par sorption, l'agent émulsionnant et le cas échéant l'huile faisant qu'il se forme non pas une masse cohérente, mais une poudre essentiellement homogène.

20. Procédé selon une des revendications 1 à 19, caractérisé en ce que l'on utilise en tant qu'agent émulsionnant au moins une des substances suivantes :
- la lécithine,
- un dérivé polyoxyéthyléné d'un ester de sorbitan,
- un dérivé d'oxyde d'éthylène de sucre ou d'ester de sucre, d'alcool de sucre et/ou d'alcool-ester de sucre.

21. Procédé selon une des revendications 12 à 20, caractérisé en ce que l'on utilise en tant que substance de nature huileuse essentiellement au moins un triglycéride.

22. Procédé selon une des revendications 1 à 21, caractérisé en ce que le dosage de la substance véhicule avec la ou les substances actives est réglé par le choix de la dimension de grains moyenne de l'amidon naturel utilisé et/ou du taux de gonflement de l'amidon ou des proportions relatives amidon/agent gonflant dans le mélange.

23. Procédé selon une des revendications 1 à 22, caractérisé en ce que la dimension de particule moyenne des substances actives encapsulées est réglée par le choix de la dimension de grain moyenne de l'amidon naturel utilisé.

24. Substance active encapsulées ou revêtues caractérisée en ce que, pour sa préparation, on utilise en tant que substance véhicule encapsulant ou revêtant un mélange d'amidon essentiellement naturel et d'au moins un agent gonflant en partie au moins l'amidon, qu'on mélange avec la substance active et au moins un agent émulsionnant pour l'encapsulation ou le revêtement.

25. Substance active encapsulée ou revêtue selon revendication 24, caractérisée en ce qu'elle consiste en une substance soluble dans l'eau ou miscible à l'eau, en particulier une vitamine soluble dans l'eau ou l'acide citrique.

26. Application du procédé selon une des revendications 1 à 23 à l'encapsulation ou au revêtement de substances actives pharmaceutiques ou cosmétiques et/ou à la préparation de médicaments ou de compositions médicinales.

27. Application du procédé selon une des revendications 1 à 23 à l'encapsulation ou au revêtement de substances solubles dans l'eau ou miscibles à l'eau, en particulier des vitamines solubles dans l'eau et/ou de l'acide citrique.

28. Application du procédé selon une des revendications 1 à 23 à l'encapsulation ou au revêtement de colles, d'arômes, de parfums, de produits de lavage, de parasiticides, d'herbicides, de colorants, d'additifs aux résines synthétiques, d'additifs pour matériaux de construction, d'additifs au béton et/ou de réactifs pour le revêtement de papiers à copier sans carbone.

29. Application du procédé selon une des revendications 1 à 23 à l'encapsulation ou au revêtement de liquides très visqueux, épais et/ou d'émulsions, tels que des aromes, des additifs pour colorants, des additifs pour résines synthétiques, des additifs pour matériaux de construction, des additifs pour béton, etc., en particulier en vue d'améliorer leurs facilités d'emploi et de dosage.
